# EUROPEAN PATENT APPLICATION

(11) **EP 4 693 315 A1**
(43) Date of publication of application: **11.02.2026**
(21) Application number: 23930704.4
(22) Date of filing: 31.03.2023
(51) Int. Cl.: G16H 10/00

(54) **DISPLAY METHOD, INFORMATION PROCESSING DEVICE, AND DISPLAY PROGRAM**

(71) Applicant: FUJITSU LIMITED, Kawasaki-shi, Kanagawa 211-8588 (JP)
(72) Inventor: TADA, Atsuko, Kawasaki-shi, Kanagawa 211-8588 (JP); AMEMIYA, Satoshi, Kawasaki-shi, Kanagawa 211-8588 (JP); MIZOUCHI, Tsuyoshi, Kawasaki-shi, Kanagawa 211-8588 (JP); OTANI, Takeshi, Kawasaki-shi, Kanagawa 211-8588 (JP); INOMATA, Akihiro, Kawasaki-shi, Kanagawa 211-8588 (JP)
(74) Representative: Hoffmann Eitle
(86) International application number: PCT/JP2023/013726
(87) International publication number: WO 2024/202070

(57) **Abstract**

A display method executed by a computer includes: displaying a path indicating a relationship diagram between services in different colors for respective types of services in a first area of a screen in a format including conditional branches; and changing a display position of the path with respect to a map such that display positions of institutions providing the services included in the path are superimposed on the map in a second area of the screen, and displaying the map and the path with the services in the different colors.

## Description

### Technical Field

The present invention relates to a display method, an information processing apparatus, and a display program.

### Background Art

As one type of workflow, a flow graph for a measure is known in which a flow of assigning an object that is a target of the measure in various fields such as medical care, nursing care, and administration, for example, a user, to a service for achieving the purpose of the measure or the like is schematized.

As one technology for supporting the evaluation of such measures, the following insurance medical data analysis system has been proposed. For example, in an insurance medical data analysis system, vector information is calculated from nationwide (first group) health and medical care (healthcare) data, and model information (prediction model) is generated from the calculated vector information and measures taken in a specific region (second group) and vector information on healthcare data for the measures. By applying healthcare data for another region (third group) to this model information, it becomes possible to predict the effectiveness of the measures taken in the specific region (second group) when taken in another region (third group), making it possible to support creation of new measures for healthcare (medical care, health checkup, or nursing care).

### Citation List

### Patent Literature

Patent Document 1: Japanese Laid-open Patent Publication No. 2021-089523

### Summary of invention

### Technical Problem

However, conventional technologies represented by the above-described insurance medical data analysis system merely predict the overall effectiveness of the measures, and therefore are not necessarily effective in verifying the effectiveness of the measures and supporting the creation of drafts of new measures.

That is, a route through services to which a user is assigned in the flow graph for the measure is not necessarily the same because it changes depending on conditional branches. For example, taking a measure in the medical field as an example, what combination of individual medical institutions cooperate with each other from the acute phase to the recovery phase until a patient returns to home depends on which medical institution the patient is assigned to at the conditional branch of each medical function in the flow graph for the measure. When the medical institutions that cooperate with each other change in this way, a difference also appears in the therapeutic effect on the patient. Nevertheless, even if the overall effect of the measure is presented, it is unclear how the effect is obtained, making it difficult to verify the effect of the measure and support creating of drafts of new measures. Furthermore, even though the above-described insurance medical data analysis system is used, it is left to a viewer to perform analysis by reading, from the flow graph for the measure, a correspondence relationship such as which part of the flow graph for the measure the cooperation between the medical institutions corresponds to. For this reason, it is difficult to improve the readability of the flow graph for the measure.

In one aspect, an object of the present invention is to provide a display method, an information processing apparatus, and a display program capable of improving visibility of service routes.

### Solution to Problem

According to an aspect of the embodiment of the invention, a display method executed by a computer, the display method includes: displaying a path indicating a relationship diagram between services in different colors for respective types of services in a first area of a screen in a format including conditional branches; and changing a display position of the path with respect to a map such that display positions of institutions providing the services included in the path are superimposed on the map in a second area of the screen, and displaying the map and the path with the services in the different colors. Advantageous Effects of Invention

According to an embodiment, visibility of service routes can be improved.

### Brief Description of Drawings

FIG. 1 is a block diagram illustrating an example of a functional configuration of a server device.
FIG. 2 is a diagram illustrating an example of a flow graph for a measure.
FIG. 3 is a diagram illustrating a specific example of a flow graph for a measure.
FIG. 4 is a diagram illustrating an example of medical institution list data.
FIG. 5 is a diagram illustrating a detailed example of a medical institution list.
FIG. 6 is a diagram (1) illustrating an example in which a care pathway is displayed.
FIG. 7 is a diagram (2) illustrating an example in which a care pathway is displayed.
FIG. 8 is a diagram (3) illustrating an example in which a care pathway is displayed.
FIG. 9 is a diagram illustrating an example of a graph of the number of days of hospital stay.
FIG. 10 is a diagram illustrating an example of a graph of hospital bed occupancy rate.
FIG. 11 is a diagram illustrating an example of a care path selection screen.
FIG. 12 is a diagram illustrating an example of a care path selection screen.
FIG. 13 is a diagram (4) illustrating an example in which a care pathway is displayed.
FIG. 14 is a diagram illustrating an example of a radar chart.
FIG. 15 is a diagram (5) illustrating an example in which a care pathway is displayed.
FIG. 16 is a diagram (6) illustrating an example in which a care pathway is displayed.
FIG. 17 is a diagram illustrating an example of a radar chart.
FIG. 18 is a diagram (7) illustrating an example in which a care pathway is displayed.
FIG. 19 is a diagram illustrating an example of a radar chart.
FIG. 20 is a schematic diagram illustrating an example of a care path prediction model.
FIG. 21 is a schematic diagram illustrating an example of an index value prediction model.
FIG. 22 is a diagram (8) illustrating an example in which a care pathway is displayed.
FIG. 23 is a diagram illustrating an example of a radar chart.
FIG. 24 is a flowchart illustrating a procedure of generation processing.
FIG. 25 is a flowchart illustrating a procedure of calculation processing.
FIG. 26 is a diagram illustrating an example of a hardware configuration.

### Description of Embodiments

Hereinafter, modes (hereinafter, described as "embodiments") for implementing a display method, an information processing apparatus, and a display program according to the present application will be described with reference to the accompanying drawings. Each embodiment merely illustrates an example or an aspect, and a numerical value, a functional range, a usage scene, and the like are not limited by such an example. Then, the embodiments can be adaptively combined to the extent that no contradiction occurs in the processing contents.

### <First Embodiment>

### <System Configuration>

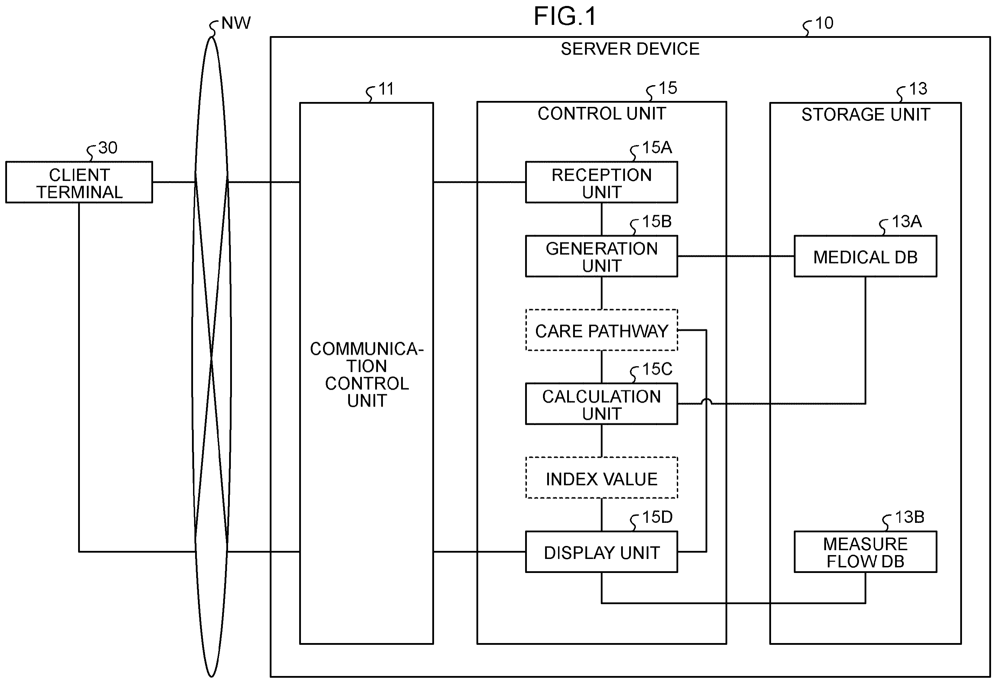

FIG. 1 is a block diagram illustrating an example of a functional configuration of a server device 10. The server device 10 illustrated in FIG. 1 provides a data base platform capable of sharing, cross-referencing, and updating flow data for measures.

For example, the server device 10 can provide the functions of the above-described data base platform as a cloud service by executing platform as a service (PaaS) type middleware or software as a service (SaaS) type applications.

As illustrated in FIG. 1, the server device 10 can be communicably connected to a client terminal 30 via a network NW. For example, the network NW may be any type of communication network, such as the Internet or a local area network (LAN), regardless of whether it is wired or wireless. Note that FIG. 1 illustrates an example in which one client terminal 30 is connected to one server device 10, but any number of client terminals 30 may be connected to one server device 10.

The client terminal 30 is a terminal device that is provided with the data base. For example, the client terminal 30 can be used by a measure planner, such as, a local government or an insurer, as an example of a person involved in implementing a measure. Furthermore, the client terminal 30 may be used by a resident or the like as an example of a service beneficiary in addition to a medical institution such as a clinic or a hospital as an example of a service provider defined in the measure. Note that, as an example, the client terminal 30 may be realized by any computer such as not only a personal computer but also a smartphone, a tablet terminal, or a wearable terminal.

### <Flow Graph for Measure>

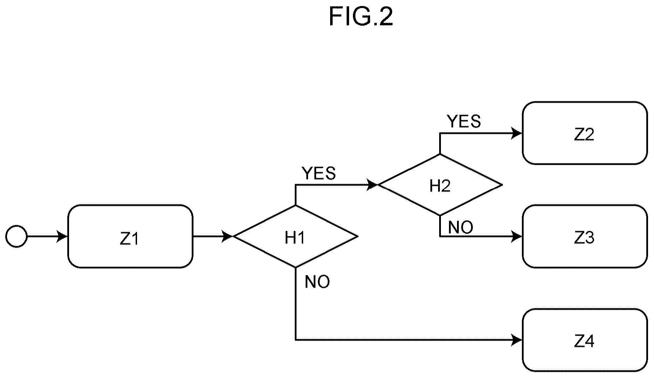

An example of a flow graph for the above-described measure is illustrated in FIG. 2. FIG. 2 is a diagram illustrating an example of a flow graph for a measure. Z1, Z2, Z3, and Z4 illustrated in FIG. 2 indicate, for example, services provided by an administrator to a user. In addition, these may be referred to as "service implementation components". Specific examples of the services include, for example, in the medical field, "intervention", such as a medical examination through a health checkup and an examination by a specialist, to which an object who is a target of the measure, such as a resident, is assigned, as well as "no intervention" such as follow-up observation, but are not limited to measures in the medical field.

H1 and H2 indicate, for example, conditional branches including conditions. In addition, these may be referred to as "conditional branch components". Specific examples of the conditions include, for example, in the medical field, an estimated glemerular filtration rate (eGFR) smaller than a threshold value, a hemoglobin A1c value (HbA1c) smaller than a threshold value, and a urinary protein value equal to or larger than a threshold value, but are not limited to conditions in the medical field.

Each of Z1, Z2, Z3, Z4, H1, and H2 may be referred to as a "component". Such a "component" may correspond to an example of a "node" in terms of graph data. Furthermore, a connection between nodes may correspond to an example of an "edge" including an "oriented edge" and the like.

Note that, in the present embodiment, measure planning in the medical field will be described as an example, but the present invention is not limited thereto. The above-described embodiment may be used for planning various measures such as tasks, tests, and questionnaires having conditional branches. In this case as well, the same effects as those of the above-described embodiment can be obtained.

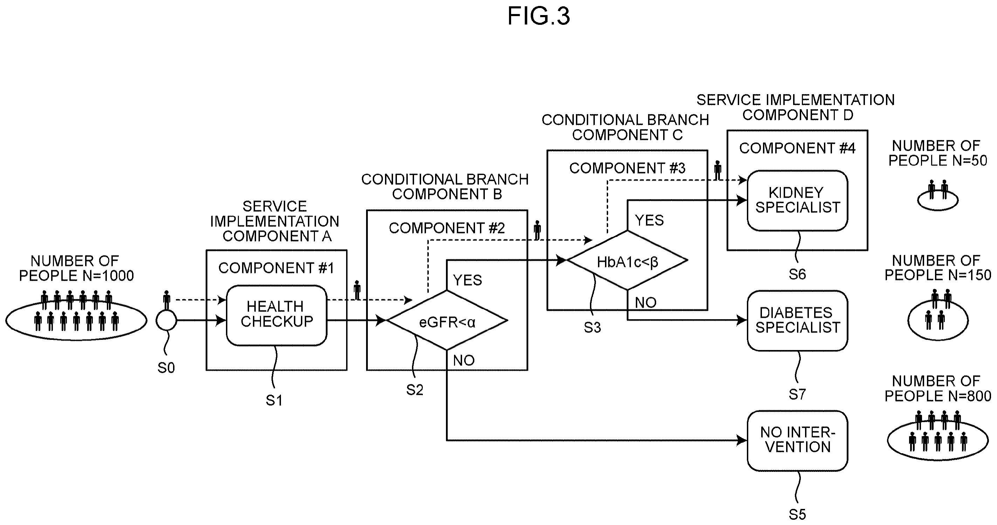

FIG. 3 is a diagram illustrating a specific example of a flow graph for a measure. As illustrated in FIG. 3, a measure is modeled as a workflow including a combination of conditional branch components, service implementation components, and the like. Then, the number of people who receive each service is output from a model trained by accumulating information and parameters on the way people flow from actual values when used for each conditional branch component.

In the example illustrated in FIG. 3, the number of people N=1000 is input in reference sign S0. In reference sign S1, component #1 as service implementation component A is set to "health checkup". In reference sign S2, component #2 as conditional branch component B is set to "eGFR<α". When "eGFR<α" is not satisfied (see the NO route in reference numeral S2), it is determined that "no intervention" is made by a specialist for relevant citizens as indicated in reference numeral S5.

On the other hand, when "eGFR<α" is satisfied (see the YES route in reference numeral S2), component #3 as conditional branch component C is set to "HbA1c<β" as indicated in reference numeral S3. When "HbA1c<β" is satisfied (see the YES route in reference numeral S3), as indicated in reference numeral S6, component #4 as conditional branch component D is set to "kidney specialist", and it is determined that the intervention of "kidney specialist" is necessary for relevant citizens. On the other hand, when "HbA1c<β" is not satisfied (see the NO route in reference numeral S3), as indicated in reference numeral S7, it is determined that the intervention of "diabetes specialist" is necessary for relevant citizens.

In the example illustrated in FIG. 3, as indicated by arrows, the numbers of people flowing to components #1, #2, #3, and #4 in this order are predicted. For example, in the flow graph for the measure illustrated in FIG. 3, the result of assigning the number of people N=1000 to interventions Z2 to Z4 is as follows. 50 people are assigned to intervention Z2. 150 people are assigned to intervention Z3. Furthermore, 800 people are assigned to intervention Z4.

Hereinafter, the example of the flow graph for the measure illustrated in FIG. 2 or 3 may be abbreviated as a "measure flow".

### <Data Base>

In the above-described data base, the measure flow may be shared in any framework. As just one example, the above-described data base can enable organizations around the world, for example, public organizations such as local governments, to share a measure flow.

Through the client terminal 30, the measure planner can refer to templates of existing measures from around the world collected in the above-described data base. For example, a draft can be updated by incorporating all or some of the existing measures similar to the draft among the templates collected in the data base.

As described above, when planning measures, it is important whether or not measures similar to the past ones have been taken, from the viewpoint of administrative (political) easiness of implementation. For this reason, it is becoming increasingly important to compare a flow graph for a draft of a measure with a flow graph for an existing measure that serves as a reference.

### <Configuration of Server Device 10>

FIG. 1 schematically illustrates blocks related to the data base included in the server device 10. As illustrated in FIG. 1, the server device 10 includes a communication control unit 11, a storage unit 13, and a control unit 15. Note that FIG. 1 merely illustrates excerpted functional units related to the above-described data base, and the server device 10 may include functional units other than those illustrated.

The communication control unit 11 is a functional unit that controls communication with other devices such as the client terminal 30. As just one example, the communication control unit 11 can be realized by a network interface card such as a LAN card. As one aspect, the communication control unit 11 receives various requests from the client terminal 30 or outputs responses to the requests to the client terminal 30.

The storage unit 13 is a functional unit that stores various types of data. As just one example, the storage unit 13 is realized by an internal, external, or auxiliary storage of the server device 10. For example, the storage unit 13 stores a medical database (DB) 13A and a measure DB 13B. Note that the medical DB 13A and the measure DB will be described together with a scene where reference, generation, or registration of the medical DB 13A and the measure DB is executed.

The control unit 15 is a functional unit that performs overall control of the server device 10. For example, the control unit 15 can be realized by a hardware processor. Alternatively, the control unit 15 may be realized by hard-wired logic. As illustrated in FIG. 1, the control unit 15 includes a reception unit 15A, a generation unit 15B, a calculation unit 15C, and a display unit 15D.

The reception unit 15A is a processing unit that receives various requests from the client terminal 30. As one aspect, the reception unit 15A can receive a care pathway display request from the client terminal 30. The term "care pathway" as used herein refers to a path indicating a route through medical institutions for medical functions when a plurality of users use medical services corresponding to the medical functions.

When receiving such a display request, the reception unit 15A can receive a designation of a data range to be used for generating a care pathway. For example, examples of items related to the data range include "patient's residential area", "period", "disease", "hospital", etc. Among them, as an example of the "patient's residential area", as well as a classification corresponding to a primary medical area such as a local government such as a municipality, a classification corresponding to a secondary medical area or a tertiary medical area in which local governments are organized may be designated. Furthermore, as an example of the "period", a designation of one or more years or the number of years, a designation of two start and end time points, or a designation of either a start or end time point and a time length can be received. In addition, as an example of the "disease", a designation of any disease such as acute myocardial infarction or cerebral infarction can be received. Furthermore, as an example of the "hospital", a designation of a name, identification information, or the like of each medical institution can be received. Although an example has been given here in which the data range is user-defined, the data range does not necessarily need to be user-defined, and may instead be designated by system definition.

The generation unit 15B is a processing unit that generates the care pathway using the medical DB 13A. Any set of medical data can be stored in the medical DB 13A. For example, the medical data may be a health checkup, a medical receipt, an electronic medical record, or the like. Furthermore, the medical DB 13A may be realized by diagnosis procedure combination (DPC) data, a database of information such as medical receipts and specific health checkups, a so-called national datebase (NDB), a national health insurance database, a so-called KDB, or the like.

Hereinafter, an example in which the medical DB 13A is realized by DPC data will be described as just one example, but the medical DB 13A may be realized by another database such as an NDB, a KDB, or a DB used by an electronic medical record system as described above.

As just one example, the generation unit 15B extracts medical data corresponding to the data range for which the designation has been received by the reception unit 15A from among the medical data stored in the medical DB 13A. For example, the generation unit 15B extracts medical data that satisfies the AND conditions for the designations of the items "patient's residential area", "period", "disease", and "hospital" from among the medical data stored in the medical DB 13A.

Subsequently, the generation unit 15B executes the following processing by the number of times corresponding to the number I of patients included in the medical data corresponding to the above-described data range. At this time, since different personal identifications (IDs) may be assigned to the same user by different hospitals in the DPC data, the generation unit 15B can identify a plurality of personal IDs for one user as the same person as the user. Such identification can be realized by collating values of items related to personal information such as place of residence, gender, and date of birth included in the DPC data.

That is, the generation unit 15B generates a list of medical institutions for medical functions when an i-th patient uses medical services corresponding to the medical functions, for example, hyperacute phase, acute phase, recovery phase, and maintenance phase. Hereinafter, the list of medical institutions for medical functions may be referred to as a "medical institution list". When generating such a medical institution list, the medical functions are identified from a medical treatment history included in the medical data for the i-th patient. At this time, from the aspect in which medical services corresponding to a plurality of medical functions can be provided by one hospital, each element of the medical institutions included in the medical institution list is identified by a combination of a medical function and a hospital. Then, the generation unit 15B sorts the medical institutions included in the medical institution list in time series.

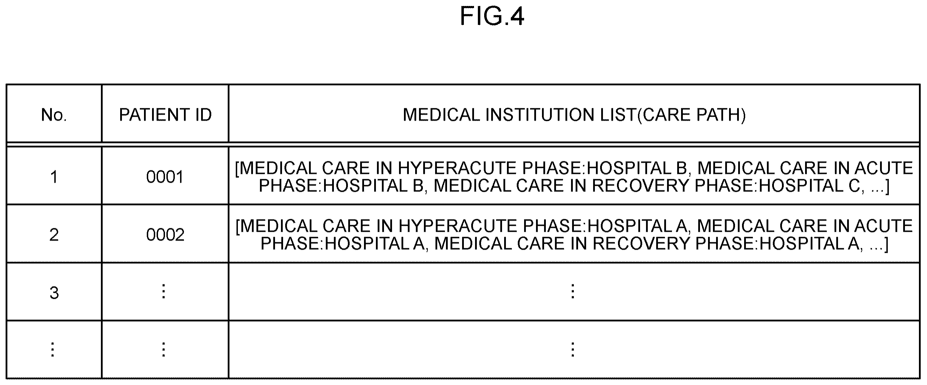

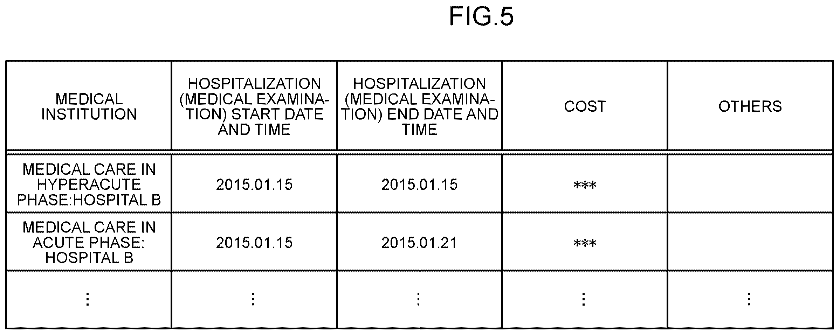

FIG. 4 is a diagram illustrating an example of medical institution list data. The medical institution list data including medical institution lists for I people is illustrated in FIG. 4. As illustrated in FIG. 4, the medical institution list data may be data in which a number for identifying a medical institution list, a patient ID for identifying a patient, and a medical institution list are associated with each other. For example, in FIG. 4, medical institution lists for patient ID "0001" and patient ID "0002" are excerpted and illustrated. Among them, the details of the medical institution list for patient ID "0001" are as illustrated in FIG. 5.

FIG. 5 is a diagram illustrating a detailed example of a medical institution list. As illustrated in FIG. 5, the medical institution list may be data in which items such as hospitalization (medical examination) start date and time when medical service provision starts, hospitalization (medical examination) end date and time when medical service provision ends, cost, and others are associated with each other for each medical institution. For example, referring to an example of an entry in the first row, it can be identified that the patient identified by patient ID "0001" had a medical examination at hospital B on January 15, 2015 for a medical service corresponding to the hyperacute phase. Furthermore, referring to an example of an entry in the second row, it can be identified that the patient identified by patient ID "0001" received a medical service corresponding to the acute phase at hospital B and was hospitalized at hospital B from January 15, 2015 to January 21, 2015.

Under a situation where such a medical institution list is generated, the generation unit 15B executes the following processing for each of the J medical institutions included in the medical institution list for the i-th patient. That is, the generation unit 15B determines whether or not no node corresponding to a j-th medical institution among the J medical institutions included in the medical institution list for the i-th patient has been generated on the care pathway being generated. Then, when no node corresponding to the j-th medical institution has been generated, the generation unit 15B adds a node corresponding to the j-th medical institution to the care pathway being generated. When a new node is added in this manner, the generation unit 15B can align the medical institutions for each medical function on the care pathway. As just one example, in a case where the classifications of the medical functions are aligned in the row direction, the generation unit 15B can arrange medical institutions corresponding to the same medical function in the same column.

Thereafter, the generation unit 15B determines whether or not no edge has been generated between the node of the j-th medical institution and the node of the j-1th medical institution in the care pathway being generated. At this time, when no edge has been generated between the node of the j-th medical institution and the node of the j-1th medical institution, the generation unit 15B adds an edge between the node of the j-th medical institution and the node of the j-1th medical institution. Further, the generation unit 15B increments the number of paths for the edge connecting the node of the j-th medical institution and the node of the j-1th medical institution, that is, the number of patients, by one.

In this manner, by adding a node and an edge for each of the J medical institutions included in the medical institution list for the i-th patient, a care path for the i-th patient is generated. Furthermore, by generating a care path for the medical institution list generated for each of the I patients, a care pathway in which the care paths for the I patients are combined is generated.

The calculation unit 15C is a processing unit that calculates an index value related to any evaluation item. Examples of such evaluation items include the number of days of hospital stay (the number of days of hospitalization), a hospital bed occupancy rate, a barthel index (BI) score difference, a resource utilization rate, and a medical expense.

As just one example, the calculation unit 15C calculates an index value for each of the K evaluation items in the minimum unit corresponding to the evaluation item. That is, the calculation unit 15C calculates an index value of a k-th evaluation item for an m-th minimum unit.

Among the above-described evaluation items, the number of days of hospital stay, the BI score difference, and the medical expense can take unique index values for each medical institution and each patient, and thus, a combination of a "node" of a medical institution included in the care pathway and a "patient" passing through the node of the medical institution is set as a minimum unit. For example, referring to an example of the number of days of hospital stay, a medical institution list for a patient who is a target of calculation among the I medical institution lists is targeted, and a difference between a hospitalization start date and time and a hospitalization end date and time for a medical institution that is a target of calculation in the medical institution list for the patient is calculated as the number of days of hospital stay. In addition, taking the BI score difference as an example, a difference between a BI score at the time point of the hospitalization start date and time and a BI score at the time point of the hospitalization end date and time is calculated as the BI score difference. Furthermore, referring to an example of the medical expense, a medical institution list for patient who is a target of calculation among the I medical institution lists is targeted, and a cost for a medical institution that is a target of calculation in the medical institution list for the patient is calculated as the medical expense.

In addition, among the above-described evaluation items, the hospital bed occupancy rate and the resource utilization rate can take unique index values for each medical institution, and thus, a "node" of a medical institution included in the care pathway is set as a minimum unit. For example, referring to an example of the hospital bed occupancy rate, the hospital bed occupancy rate is calculated by dividing the aggregate value of patients who receive medical services provided by a medical institution that is a target of calculation for each section in which the data range of the care pathway is divided, such as "day", "week", or "month", by the number of hospital beds owned by the medical institution. Furthermore, referring to an example of the resource utilization rate, the resource utilization rate is calculated by dividing the aggregate value of patients who receive medical services provided by a medical institution that is a target of calculation for each of the above sections by the number of medical personnel belonging to the medical institution or the number of medical personnel attending the medical institution. Note that the classification of "medical personnel" mentioned here can be doctors only, nurses only, or both doctors and nurses.

After the index value for the minimum unit is calculated in this manner, the calculation unit 15C calculates an index value of the k-th evaluation item for the entire care pathway. For example, the calculation unit 15C calculates a statistical value, such as an average value, a median value, a maximum value, or a minimum value, of index values calculated for each minimum unit of combination of "medical institution" and "patient" or for each minimum unit of "medical institution".

The display unit 15D is a processing unit that displays various types of information on the client terminal 30. As one aspect, the display unit 15D can cause the client terminal 30 to display a care pathway generated by the generation unit 15B.

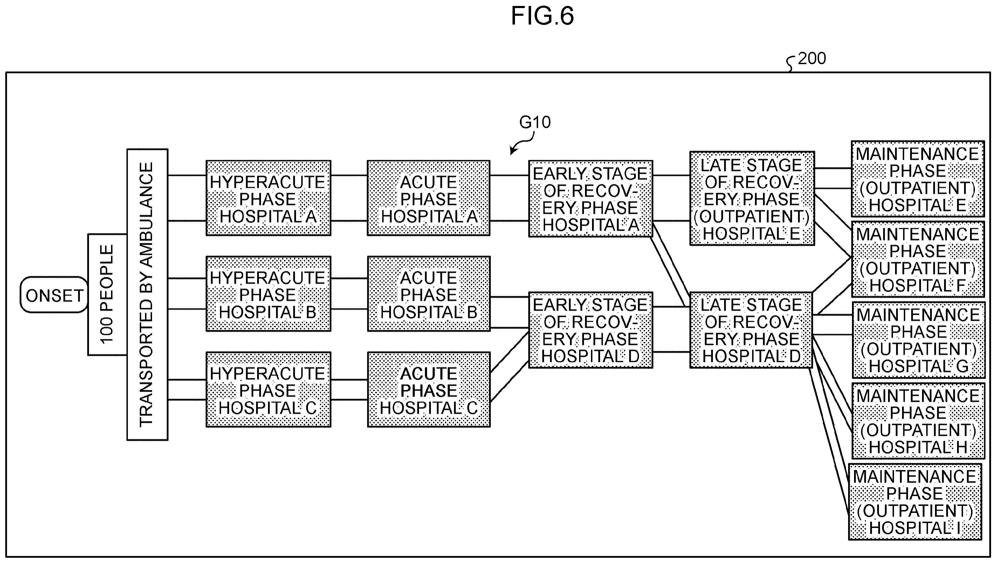

FIG. 6 is a diagram (1) illustrating an example in which a care pathway is displayed. FIG. 6 illustrates an example of a screen 200 including a care pathway G10 in which care paths for 100 patients who have been transported by ambulance are combined as a display for a measure planner. By displaying such a care pathway G10, it is possible to visualize the state of cooperation between medical functions. For example, according to the care pathway G10, it can be grasped that hospital A, hospital B, and hospital C provide medical care in the hyperacute phase and the acute phase independently of each other. Furthermore, it can be grasped that hospital B, hospital C, and hospital D cooperate with each other in the medical care from the acute phase to the early stage of the recovery phase. Furthermore, it can be grasped that hospital A, hospital D, and hospital E cooperate with each other in the medical care from the early stage of the recovery phase to the late stage of the recovery phase. Furthermore, it can be grasped that hospital E and the hospital F cooperate with each other and hospital D and hospitals F to I cooperate with each other in the medical care from the late stage of the recovery phase to the maintenance phase.

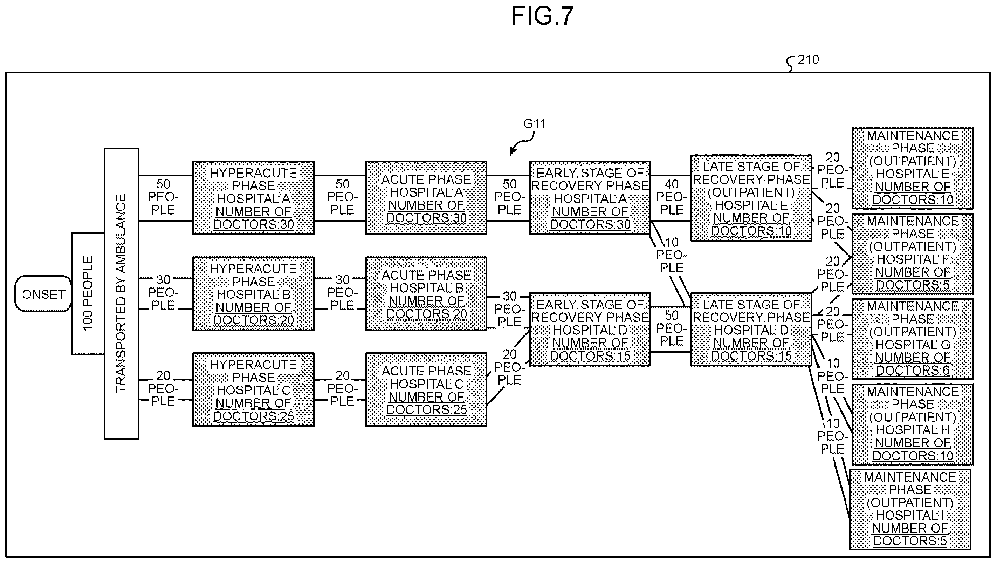

FIG. 7 is a diagram (2) illustrating an example in which a care pathway is displayed. FIG. 7 also illustrates an example of a screen 210 including a care pathway G11 in which care paths for 100 patients who have been transported by ambulance are combined as a display for a measure planner. As illustrated in FIG. 7, in the care pathway G11 compared with the care pathway G10 illustrated in FIG. 6, the number of patients passing through each edge included in the care pathway G11 is plotted. Furthermore, in the care pathway G11 compared with the care pathway G10 illustrated in FIG. 6, each edge included in the care pathway G11 is displayed with a thickness corresponding to the number of patients passing through the edge. In addition, in the care pathway G11, resources at each node included in the care pathway G11, for example, the number of doctors, are plotted. By displaying such a care pathway G11, it is possible to visualize the balance between demand and supply at each medical institution.

Furthermore, the display unit 15D can also display an index value of an evaluation item calculated by the calculation unit 15C in association with the medical institution included in the care pathway generated by the generation unit 15B. At this time, the display unit 15D can display index values of evaluation items for all the medical institutions included in the care pathway in association with the medical institutions, but can also narrow down the evaluation items to evaluation items of which the index values satisfy a specific condition and display the index values of the narrowed-down evaluation items in association with the medical institutions.

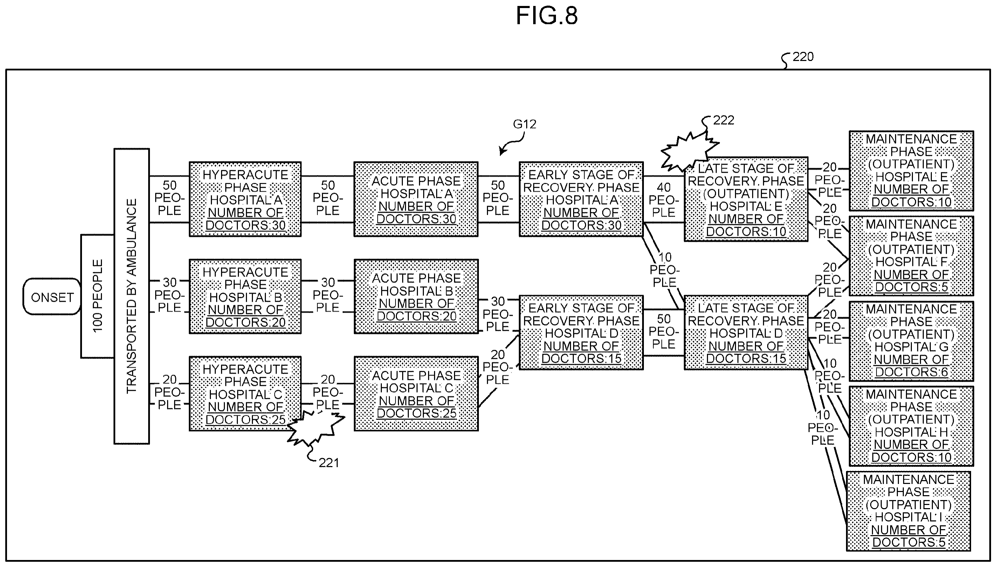

FIG. 8 is a diagram (3) illustrating an example in which a care pathway is displayed. FIG. 8 also illustrates an example of a screen 220 including a care pathway G12 in which care paths for 100 patients who have been transported by ambulance are combined as a display for a measure planner. As illustrated in FIG. 8, the care pathway G12 is different from the care pathway G11 illustrated in FIG. 7 in that a medical institution satisfying condition 1 that the number of patients who have stayed at the hospital for days exceeding a first threshold value Th1 is equal to or greater than a second threshold value Th2 is highlighted. As an example, the first threshold value Th1 to be compared with the number of days of hospital stay can be set based on a statistical value, such as an average value, a median value, or a standard deviation, of the numbers of days of hospital stay of patients who receive medical service for the same disease at the same medical institution. For example, in the care pathway G12, an alert 221 is displayed in association with the node "hospital C for medical care in hyperacute phase". By displaying such an alert 221, an occurrence of stay (prolonged hospitalization) can be visualized.

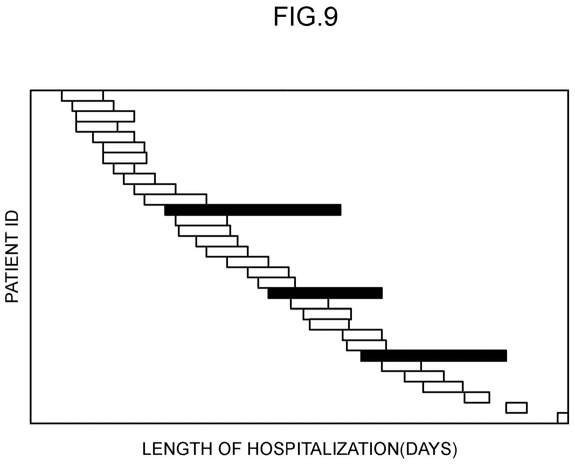

For example, when an operation on the alert 221 of the node "hospital C for medical care in hyperacute phase" of the care pathway G12 is received, a graph of the number of days of hospital stay for the node "hospital C for medical care in hyperacute phase" (see FIG. 9) can be displayed.

FIG. 9 is a diagram illustrating an example of a graph of the number of days of hospital stay. The vertical axis of the graph illustrated in FIG. 9 corresponds to the patient ID, and the horizontal axis of the graph corresponds to the number of days of hospital stay (days). As illustrated in FIG. **9****,** in the graph of the number of days of hospital stay, plots of the numbers of days of hospital stay of patients who have stayed at the hospital for days exceeding the first threshold value among patients who have received medical services at hospital C for medical care in hyperacute phase are highlighted in black. By displaying such a graph of the number of days of hospital stay, it is possible to grasp a patient who causes an occurrence of stay (prolonged hospitalization) at the node "hospital C for medical care in hyperacute phase" and the number of days of hospital stay.

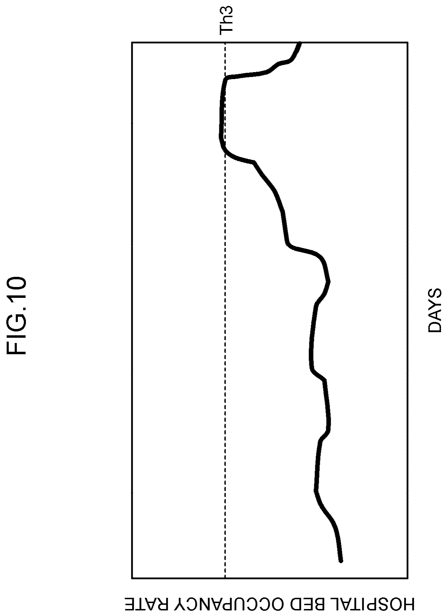

Furthermore, the care pathway G12 illustrated in FIG. 8 is different from the care pathway G11 illustrated in FIG. 7 in that medical institutions satisfying condition 2 that the hospital bed occupancy rate exceeds the third threshold Th3 are highlighted. For example, in the care pathway G12, an alert 222 is displayed in association with the node "hospital E for medical care in late stage of recovery phase". When an operation on such an alert 222 is received, a graph of hospital bed occupancy rate for the node "hospital E for medical care in late stage of recovery phase" (see FIG. 10) can be displayed.

FIG. 10 is a diagram illustrating an example of a graph of hospital bed occupancy rate. The vertical axis of the graph illustrated in FIG. 10 corresponds to the hospital bed occupancy rate, and the horizontal axis of the graph corresponds to the days. As illustrated in FIG. 10, in the graph of hospital bed occupancy rate, a trend in hospital bed occupancy rate calculated by the calculation unit 15C for each section, for example, each "day", that is, time-series data, is displayed. By displaying such a graph of hospital bed occupancy rate, it is possible to grasp the time when the hospital bed occupancy rate reaches the third threshold Th3 at the node "hospital E for medical care in late stage of recovery phase".

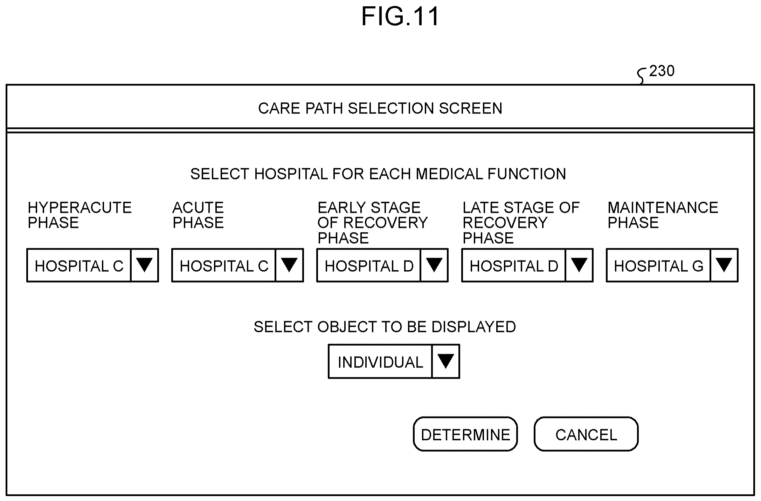

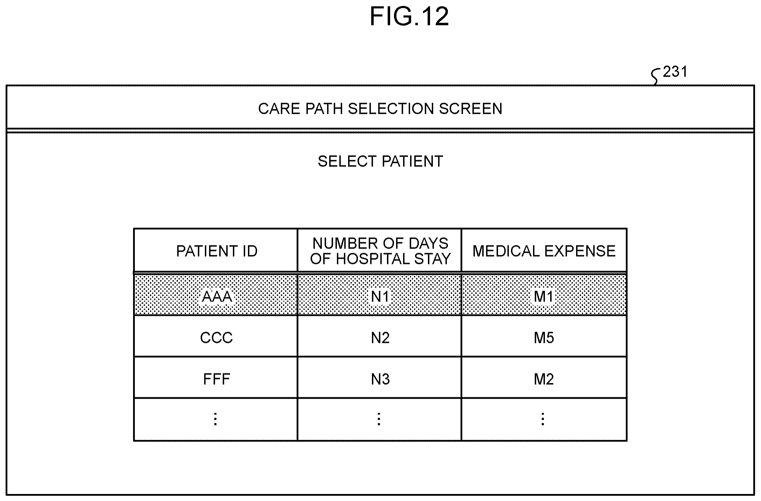

In another aspect, the display unit 15D can display a care path for a specific user to be superimposed on the care pathway generated by the generation unit 15B. In this case, the display unit 15D can receive a designation of a specific user via a care path selection screen illustrated in FIG. 11 or 12. Note that the care path selection screen illustrated in FIG. 11 or 12 can be displayed either after a care pathway is displayed or when a care pathway display request is received.

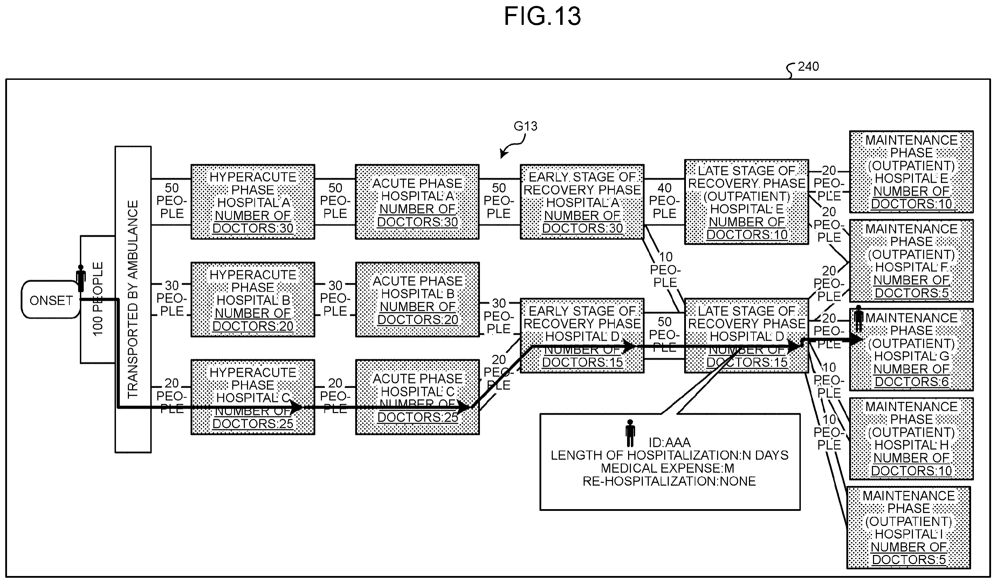

FIGS. 11 and 12 are diagrams each illustrating an example of a care path selection screen. As illustrated in FIG. 11, on the care path selection screen 230, a pull-down menu is illustrated as an example of a graphical user interface (GUI) for each medical function for designating a medical institution in charge of medical care for the medical function. For example, referring to the example illustrated in FIG. 11, an example is illustrated in which a care path for "hospital C" in the hyperacute phase, "hospital C" in the acute phase, "hospital D" in the early stage of the recovery phase, "hospital D" in the late stage of the recovery phase, and "hospital G" in the maintenance phase. When a determination button is operated in a state where the care path is selected in this manner, as illustrated in FIG. 12, a patient list L1 in which patients corresponding to the care path illustrated in FIG. 11 are listed is displayed. By receiving a selection of a patient from among the patients included in the patient list L1, a care path for a specific user can be selected. For example, in a case where a patient whose patient ID is "AAA" is selected among the patients included in the patient list L1, a screen 240 including a care pathway G13 illustrated in FIG. 13 is displayed.

FIG. 13 is a diagram (4) illustrating an example in which a care pathway is displayed. FIG. 13 also illustrates an example of a screen 240 including a care pathway G13 in which care paths for 100 patients who have been transported by ambulance are combined as a display for a measure planner. As illustrated in FIG. 13, in the care pathway G13 compared with the care pathway G11 illustrated in FIG. 7, a care path for the patient ID "AAA", for example, an arrow of a thick solid line in the diagram, is displayed in a superimposed manner. That is, as the care path for the patient ID "AAA", a route including a node "hospital C for medical care in hyperacute phase", a node "hospital C for medical care in acute phase", a node "hospital D for medical care in early stage of recovery phase", a node "hospital D for medical care in early stage of recovery phase", and a node "hospital G for medical care in maintenance phase" is displayed. Furthermore, in the care path for the patient ID "AAA", the length of hospitalization "N days" in the entire care path, the medical expense "M" in the entire care path, and the re-hospitalization "none" are displayed in association with each other.

Furthermore, a statistical index value of each evaluation item for the entire measure flow (the care pathway G13) and an index value of each evaluation item for a specific user, for example, the patient ID "AAA", can be displayed in association with each other on the care pathway G13 illustrated in FIG. 13.

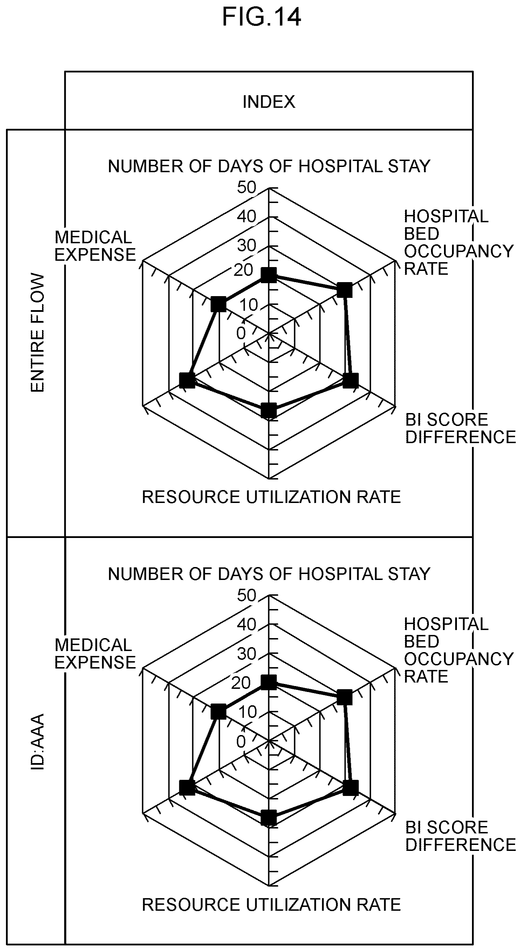

FIG. 14 is a diagram illustrating an example of a radar chart. FIG. 14 illustrates a radar chart in which statistical index values of five evaluation items for the entire measure flow (the care pathway G13) are plotted, and a radar chart in which statistical index values of five evaluation items for the patient ID "AAA" are plotted. Among them, the statistical index value of each evaluation item for the entire measure flow can be obtained by calculating a statistical value, for example, an average value, of index values for I patients, that is, 100 patients in this example, in the individual entire care paths for each evaluation item. By displaying the radar chart for the entire measure flow and the radar chart for the specific user, relative evaluations of index values can be performed between the entire measure flow and the care path for the specific user. For example, when the care path for the specific user is superior to the entire measure flow, a draft of a measure for leaving the care path at the time of reorganization of medical care can be created. Furthermore, when the care path for the specific user is inferior to the entire measure flow, a draft of a measure for removing the care path at the time of reorganization of medical care can be created.

Not limited to such a specific user, the display unit 15D can also display a designated care path in a superimposed manner on the care pathway generated by the generation unit 15B. For example, the display unit 15D can display the care path selected via the care path selection screen 230 illustrated in FIG. 11 in a superimposed manner on the care pathway generated by the generation unit 15B.

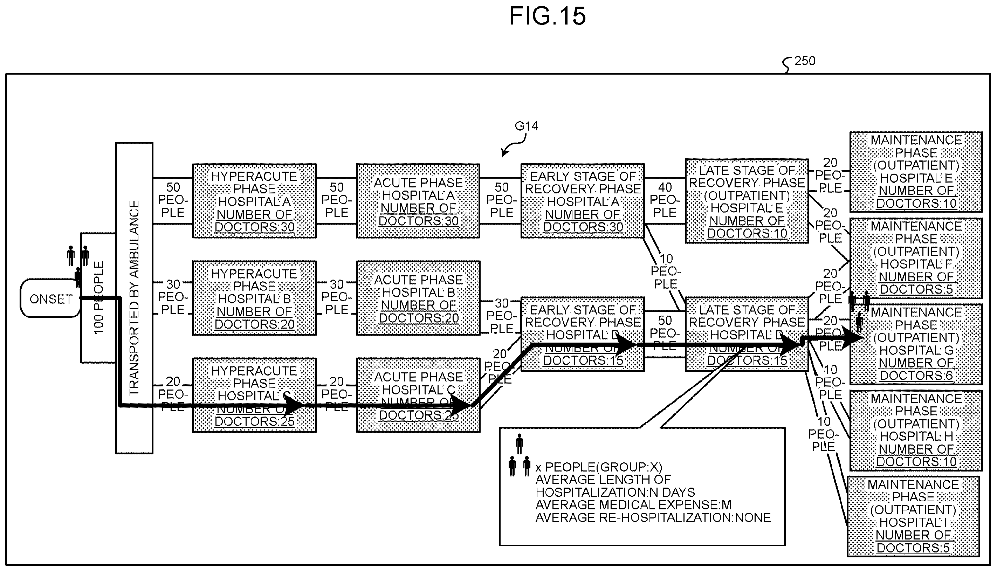

FIG. 15 is a diagram (5) illustrating an example in which a care pathway is displayed. FIG. 13 also illustrates an example of a screen 250 including a care pathway G14, in which care paths for 100 patients who have been transported by ambulance are combined as a display for a measure planner. As illustrated in FIG. 15, the care pathway G14 is identical to the care pathway G13 illustrated in FIG. 13 in that a designated care path, for example, an arrow of a thick solid line in the diagram, is displayed in a superimposed manner. That is, a care path including a node "hospital C for medical care in hyperacute phase", a node "hospital C for medical care in acute phase", a node "hospital D for medical care in early stage of recovery phase", a node "hospital D for medical care in early stage of recovery phase", and a node "hospital G for medical care in maintenance phase" is displayed. On the other hand, the care pathway G14 is different from the care pathway G13 illustrated in FIG. 13 in that, instead of the care path for the patient ID "AAA", a care path for group X of x patients belonging to the care path is displayed in a superimposed manner. Furthermore, in the care path for group X of x patients, the average length of hospitalization "N days" in the entire care path, the average medical expense "M" in the entire care path, and the average re-hospitalization "none" for group X are displayed in association with each other.

Furthermore, a statistical index value of each evaluation item for the entire measure flow (the care pathway G14) and a statistical index value of each evaluation item for a user group of group X can be displayed in association with each other on the care pathway G14 illustrated in FIG. 15.

For example, a radar chart in which statistical index values of five evaluation items for the entire measure flow (the care pathway G14) are plotted and a radar chart in which statistical index values of five evaluation items for the user group of group X are plotted can be displayed. Among them, the statistical index value of each evaluation item for the entire measure flow can be obtained by calculating a statistical value, for example, an average value, of index values for I patients, that is, 100 patients in this example, in the individual entire care paths for each evaluation item. On the other hand, the statistical index value of each evaluation item for the user group of group X can be obtained by calculating a statistical value, for example, an average value, of index values for the user group of group X belonging to the care path including the node "hospital C for medical care in hyperacute phase", the node "hospital C for medical care in acute phase", the node "hospital D for medical care in early stage of recovery phase", the node "hospital D for medical care in early stage of recovery phase", and the node "hospital G for medical care in maintenance phase". By displaying the radar chart for the entire measure flow and the radar chart for group X belonging to the specific care path, it is possible to consider whether or not it is better to increase the number of members in group X.

In a further aspect, the display unit 15D can display a specific measure flow among the measure flows included in the measure DB 13B and a care pathway generated in a data range corresponding to the specific measure flow in association with each other. At this time, the display unit 15D can display nodes corresponding to the same type of medical function between the measure flow and the care pathway in a common display format, for example, in a common color or in a common hatching manner. Furthermore, the display unit 15D can display the nodes of the medical institutions included in the care pathway in association with the locations of the medical institutions on the map.

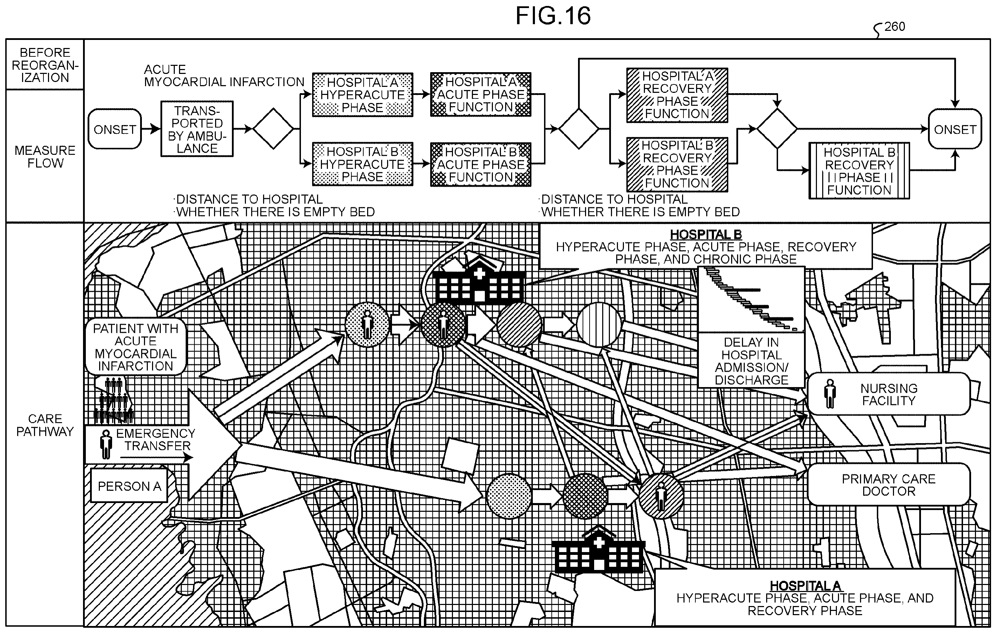

FIG. 16 is a diagram (6) illustrating an example in which a care pathway is displayed. In FIG. 16, as just one example, a screen 260 including a measure flow before reorganization of medical care and a care pathway generated in a corresponding data range before the reorganization of medical care is displayed. Furthermore, in FIG. 16, a care pathway for a patient with acute myocardial infarction as an example of a disease is displayed. Furthermore, in FIG. 16, a care pathway in which hospital A and hospital B are designated as an example of a data range is displayed.

As illustrated in FIG. 16, between the measure flow before the reorganization of medical care and the care pathway, a node "hospital A for medical care in hyperacute phase" and a node "hospital B for medical care in hyperacute phase" are displayed in a common hatching manner, that is, in a light dotted hatching manner in the diagram. Furthermore, between the measure flow before the reorganization of medical care and the care pathway, a node "hospital A for medical care in acute phase" and a node "hospital B for medical care in acute phase" are displayed in a common hatching manner, that is, in a dark dotted hatching manner in the diagram. Furthermore, between the measure flow before the reorganization of medical care and the care pathway, a node "hospital A for medical care in recovery phase" and a node "hospital B for medical care in recovery phase" are displayed in a common hatching manner, that is, in a right-upwardly oblique hatching manner in the diagram. Furthermore, between the measure flow before the reorganization of medical care and the care pathway, a node "hospital B for medical care in chronic phase" is displayed in a common hatching manner, that is, in a vertical hatching manner in the diagram.

In addition, in the care pathway illustrated in FIG. 16, a node "hospital A for medical care in hyperacute phase", a node "hospital A for medical care in acute phase", and a node "hospital A for medical care in recovery phase" are arranged in association with an icon for the location of hospital A on the map. Furthermore, in the care pathway illustrated in FIG. 16, a node "hospital B for medical care in hyperacute phase", a node "hospital B for medical care in acute phase", a node "hospital B for medical care in recovery phase", and a node "hospital B for medical care in chronic phase" are arranged in association with an icon for the location of hospital B on the map.

Additionally, in the care pathway illustrated in FIG. 16, a care path for a specific user "A" is displayed in a superimposed manner, and a graph of the number of days of hospital stay is displayed in association with an edge where stay has occurred.

Here, a statistical index value of each evaluation item for the entire measure flow and an index value of each evaluation item for a specific hospital, for example, "hospital A" and "hospital B", can be displayed in association with each other on the care pathway illustrated in FIG. 16.

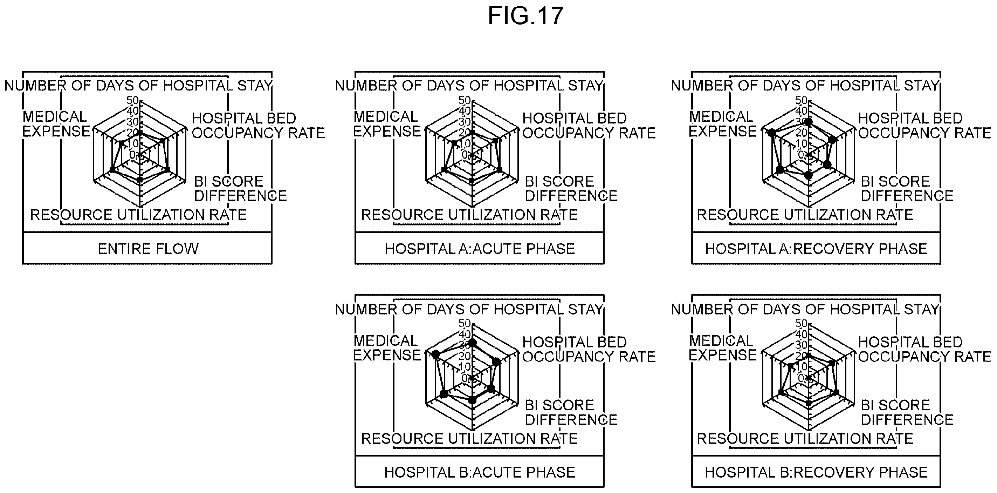

FIG. 17 is a diagram illustrating an example of a radar chart. FIG. 17 illustrates a radar chart in which statistical index values of five evaluation items for the entire measure flow before organization of medical care are plotted, a radar chart in which statistical index values of five evaluation items for hospital A are plotted, and a radar chart in which statistical index values of five evaluation items for hospital B are plotted. For these hospitals A and B, radar charts in which statistical index values of five evaluation items are plotted for the medical functions "acute phase" and "recovery phase", respectively, may be displayed. By displaying these radar charts, the statistical index values of the respective evaluation items for the same medical function between hospital A and hospital B can be compared, which can be used as a determination material such as leaving the medical function of the hospital with a better evaluation in the evaluation item on which the measure planner places importance in the reorganization of medical care, or eliminating the medical function of the hospital with a worse evaluation in the evaluation item on which the measure planner places importance in the reorganization of medical care.

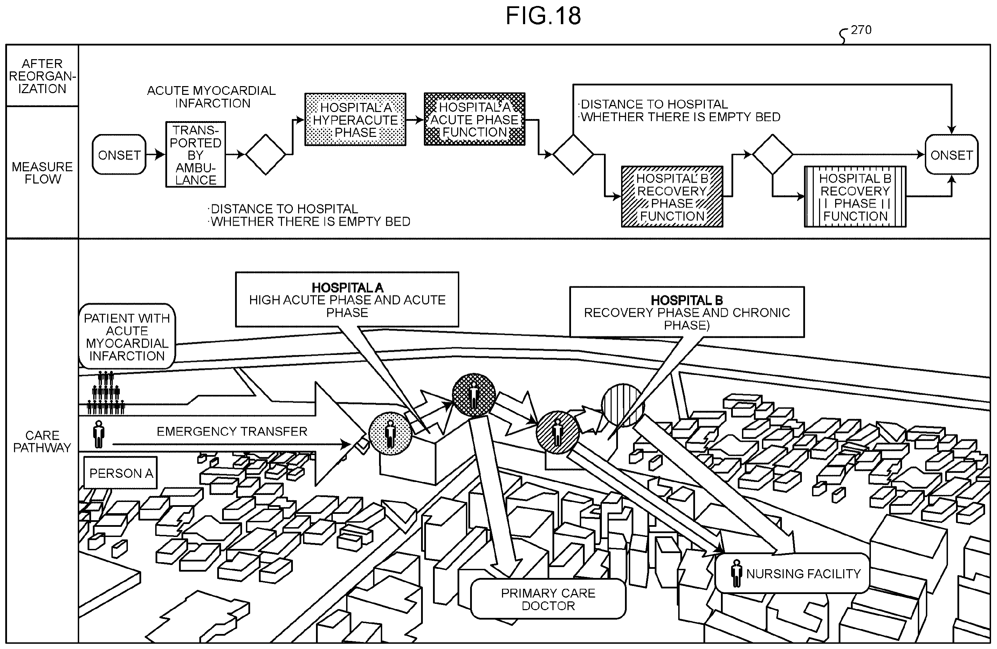

FIG. 18 is a diagram (7) illustrating an example in which a care pathway is displayed. In FIG. 18, as just one example, a screen 270 including a measure flow after reorganization of medical care and a care pathway generated in a corresponding data range after the reorganization of medical care is displayed. Furthermore, in FIG. 18, a care pathway for a patient with acute myocardial infarction as an example of a disease is displayed. Furthermore, in FIG. 18, a care pathway in which hospital A and hospital B are designated as an example of a data range is displayed.

The measure flow after the reorganization of medical care illustrated in FIG. 18 is different from the measure flow before the reorganization of medical care illustrated in FIG. 16 in that the node "hospital B for medical care in hyperacute phase" and the node "hospital B for medical care in acute phase" are eliminated, and the node "hospital A for medical care in recovery phase" is eliminated.

As illustrated in FIG. 18, between the measure flow after the reorganization of medical care and the care pathway, a node "hospital A for medical care in hyperacute phase" is displayed in a common hatching manner, that is, in a light dotted hatching manner in the diagram. Furthermore, between the measure flow after the reorganization of medical care and the care pathway, a node "hospital A for medical care in acute phase" is displayed in a common hatching manner, that is, in a dark dotted hatching manner in the diagram. Furthermore, between the measure flow after the reorganization of medical care and the care pathway, a node "hospital B for medical care in recovery phase" is displayed in a common hatching manner, that is, in a right-upwardly oblique hatching manner in the diagram. Furthermore, between the measure flow after the reorganization of medical care and the care pathway, a node "hospital B for medical care in chronic phase" is displayed in a common hatching manner, that is, in a vertical hatching manner in the diagram.

In addition, in the care pathway illustrated in FIG. 18, a node "hospital A for medical care in hyperacute phase" and a node "hospital A for medical care in acute phase" are arranged in association with an icon for the location of hospital A on the map. Furthermore, in the care pathway illustrated in FIG. 18, a node "hospital B for medical care in recovery phase" and a node "hospital B for medical care in chronic phase" are arranged in association with an icon for the location of hospital B on the map. Additionally, a care path for a specific user **"A" is** displayed in a superimposed manner on the care pathway illustrated in FIG. 18.

Here, a statistical index value of each evaluation item of the entire measure flow before or after the reorganization of medical care and an index value of each evaluation item for a specific hospital before or after the reorganization of medical care, for example, "hospital A" and "hospital B", can be displayed in association with each other on the care pathway illustrated in FIG. 18.

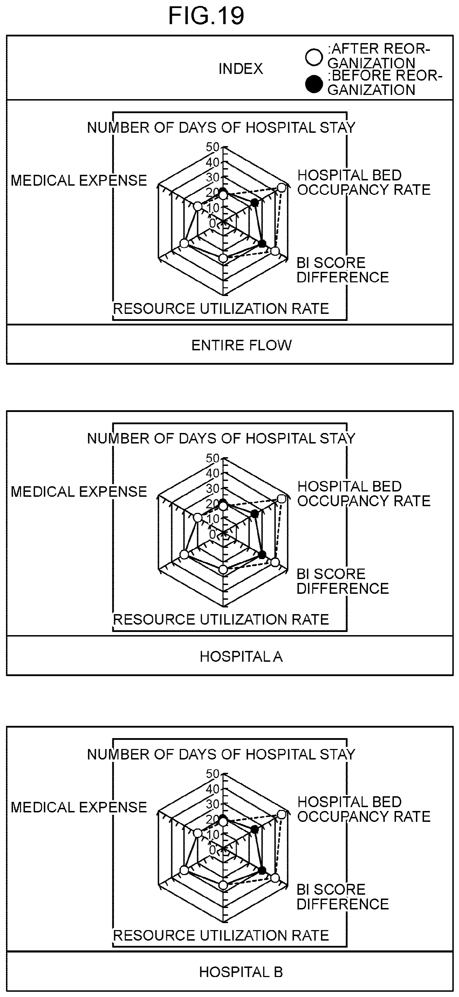

FIG. 19 is a diagram illustrating an example of a radar chart. FIG. 19 illustrates a radar chart in which statistical index values of five evaluation items for the entire measure flow before and after reorganization of medical care are plotted, a radar chart in which statistical index values of five evaluation items for hospital A before and after reorganization of medical care are plotted, and a radar chart in which statistical index values of five evaluation items for hospital B before and after reorganization of medical care are plotted. For these hospitals A and B, radar charts in which statistical index values of five evaluation items before and after reorganization of medical care are plotted for the medical functions left after the reorganization of medical care may be displayed. By displaying these radar charts, it is possible to grasp a tendency toward an improvement in each evaluation item, that is, an achievement of the reorganization of medical care, such as an increase in BI score difference, an increase in hospital bed occupancy rate within an acceptable range, and a decrease in the number of days of hospital stay in hospital A and hospital B in the entire measure flow after the reorganization of medical care.

In a further aspect, the display unit 15D can predict a care path for an individual based on the individual's attributes or personal information, and display the predicted care path for the individual in a superimposed manner on the care pathway.

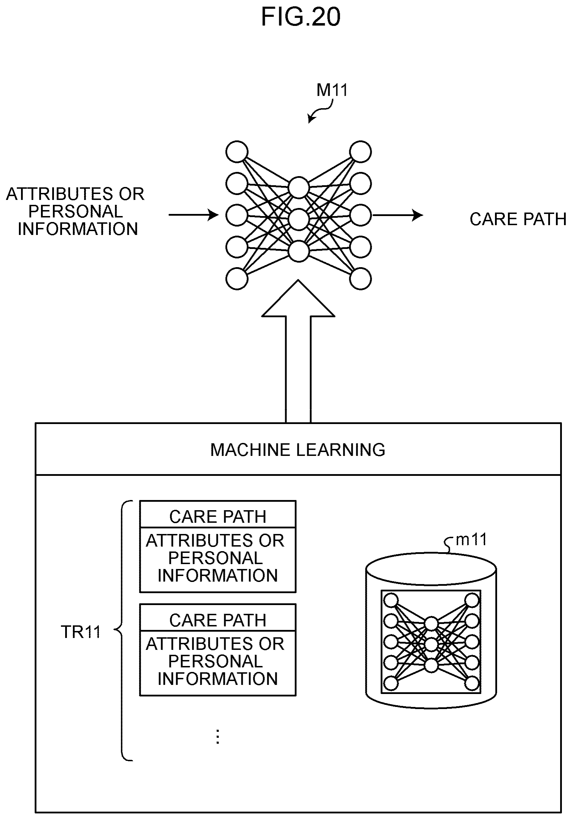

FIG. 20 is a schematic diagram illustrating an example of a care path prediction model. As illustrated in FIG. 20, a machine learning model m1 is used to predict a care path for an individual. For example, the machine learning model m1 may be realized by a neural network, a support vector machine, gradient boosting, or the like. To train such a machine learning model m1, it is possible to use a data set TR11 including training data in which attribute information such as part of address, age, and gender for an individual, or personal information such as address, age, gender, health checkup result, primary care doctor, and medical history for an individual is associated with a correct answer label for a care path.

For example, in a training phase, the machine learning model m1 can be trained according to any machine learning algorithm such as deep learning, using at least one of the attribute information or personal information for the individual as an explanatory variable of the machine learning model m1, and using the label as an objective variable of the machine learning model m1. As a result, the trained machine learning model M1 is obtained.

In a prediction phase, at least one of the attribute information or personal information for the individual is input to the machine learning model M1. The machine learning model M1 to which the attribute information or personal information for the individual is input in this manner outputs a care path for the individual. Furthermore, by generating a machine learning model M1 for each disease, a care path for any disease can be predicted.

Note that, in FIG. 20, the attribute information or the personal information for the individual is exemplified as an input to the machine learning model M1, but in addition to this, a part of the care path, for example, an end point, may be also input.

Additionally, the display unit 15D can predict an index value for the individual based on the attributes or personal information for the individual, and display the predicted index value for the individual in association with the care pathway.

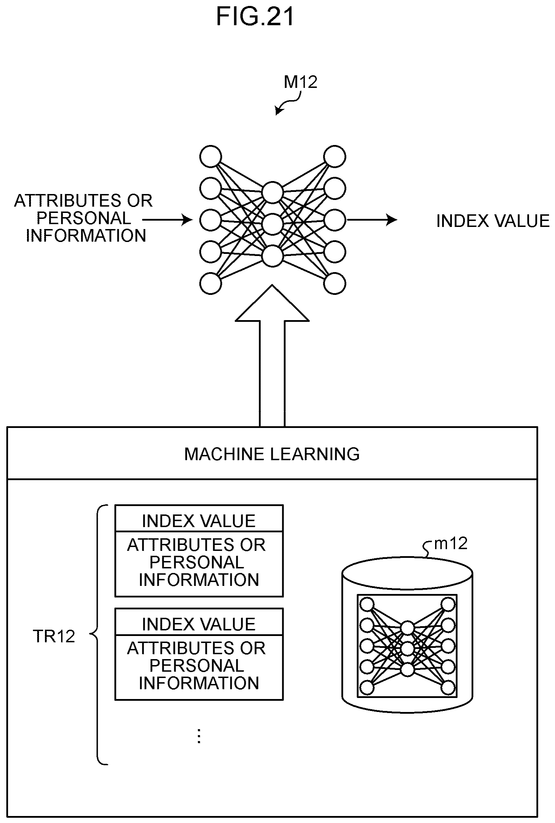

FIG. 21 is a schematic diagram illustrating an example of an index value prediction model. As illustrated in FIG. 21, a machine learning model m2 is used to predict an index value for an individual. For example, the machine learning model m2 may be realized by a neural network, a support vector machine, gradient boosting, or the like. To train such a machine learning model m2, it is possible to use a data set TR12 including training data in which attribute information such as part of address, age, and gender for an individual, or personal information such as address, age, gender, health checkup result, primary care doctor, and medical history for an individual is associated with a correct answer label for an index value of a specific evaluation item.

For example, in a training phase, the machine learning model m2 can be trained according to any machine learning algorithm such as deep learning, using at least one of the attribute information or personal information for the individual as an explanatory variable of the machine learning model m2, and using the label as an objective variable of the machine learning model m2. As a result, the trained machine learning model M2 is obtained.

In a prediction phase, at least one of the attribute information or personal information for the individual is input to the machine learning model M2. The machine learning model M2 to which the attribute information or personal information for the individual is input in this manner outputs a predicted index value for the individual. Furthermore, by generating a machine learning model M2 for each disease and for each evaluation item, a care path for any disease can be predicted.

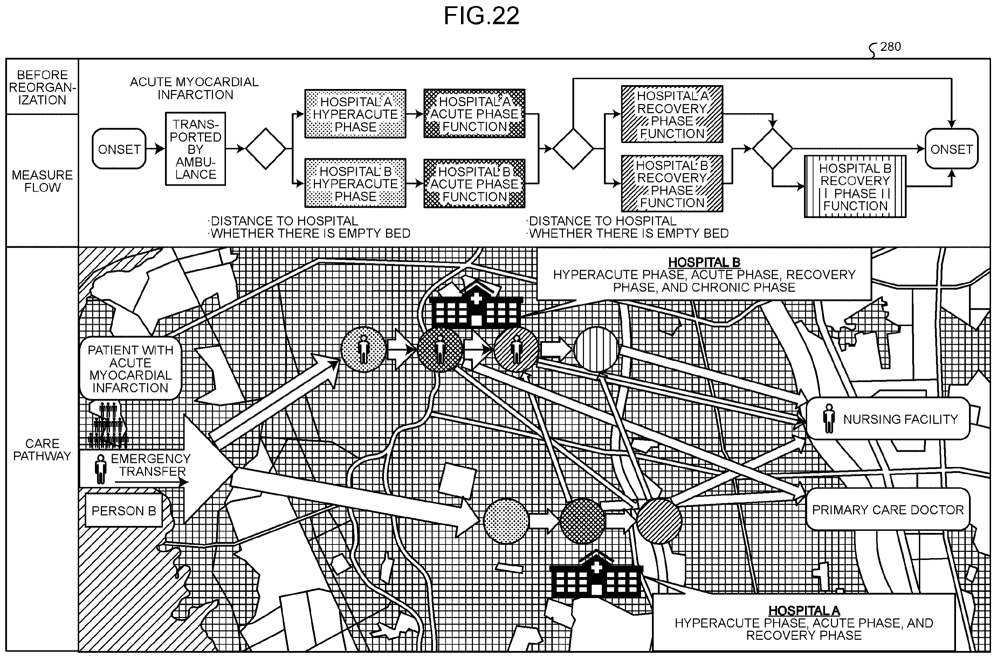

FIG. 22 is a diagram (8) illustrating an example in which a care pathway is displayed. In FIG. 18, as just one example, a screen 280 including a measure flow before reorganization of medical care and a care pathway generated in a corresponding data range before the reorganization of medical care is displayed. Furthermore, in FIG. 18, a care pathway for a patient with acute myocardial infarction as an example of a disease is displayed. Furthermore, in FIG. 16, a care pathway in which hospital A and hospital B are designated as an example of a data range is displayed.

The care pathway illustrated in FIG. 22 is different from the care pathway illustrated in FIG. 16 in that a care path for individual "B" predicted by the care path prediction model illustrated in FIG. 20 is displayed in a superimposed manner. Furthermore, a statistical index value of each evaluation item for the entire measure flow and a predicted index value of each evaluation item for the specific individual "B" can be displayed in association with each other on the care pathway illustrated in FIG. 22.

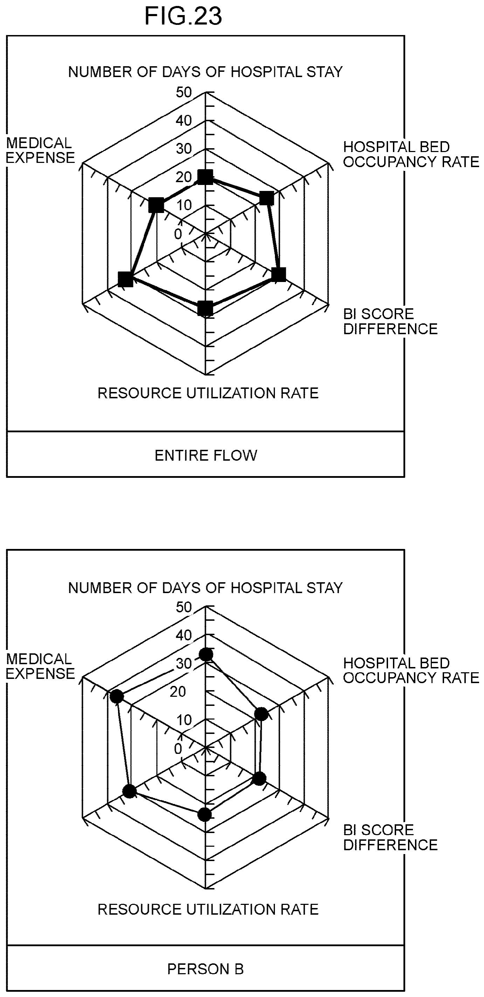

FIG. 23 is a diagram illustrating an example of a radar chart. FIG. 23 illustrates a radar chart in which statistical index values of five evaluation items for the entire measure flow before reorganization of medical care are plotted, and a radar chart in which predicted index values of five evaluation items for individual "B" are plotted. By displaying these radar charts, each index (radar chart graph) when passing through the care pathway for person B on the map can be compared with that in the entire measure flow, thereby grasping that an evaluation item, for example, a hospital stay period, for person B tends to be longer than that in the entire measure flow.

### <Flow of Processing>

Next, a flow of processing of the server device 10 according to the present embodiment will be described. Here, (1) generation processing and (2) calculation processing executed by the server device 10 will be described.

### (1) Generation Processing

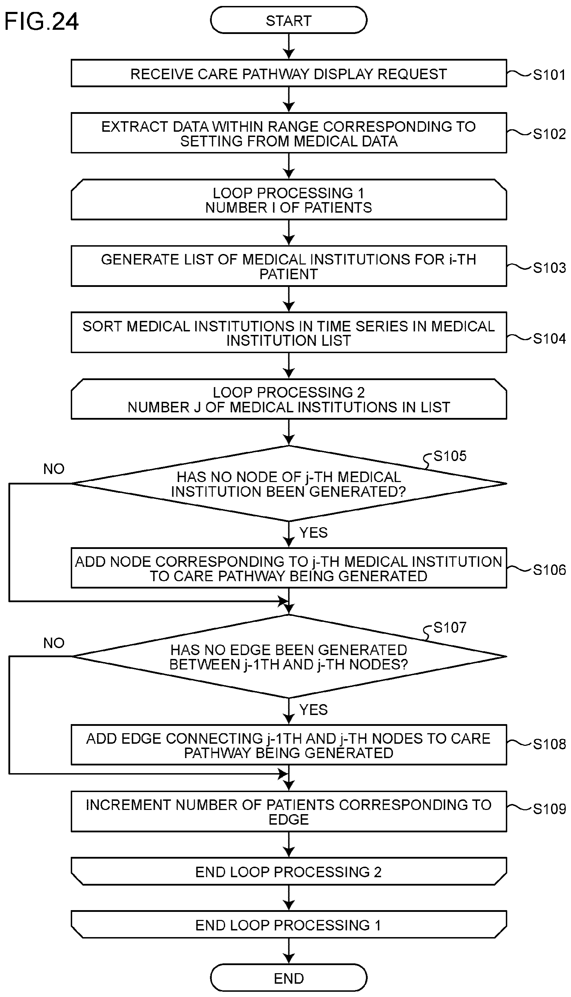

FIG. 24 is a flowchart illustrating a procedure of generation processing. As illustrated in FIG. 24, when the reception unit 15A receives a care pathway display request (step S101), the generation unit 15B executes the following processing. That is, the generation unit 15B extracts medical data corresponding to a data range designated in the display request from the medical data stored in the medical DB 13A (step S102).

Subsequently, the generation unit 15B executes loop processing 1 that repeats processing from the following steps S103 to the following step S109 by the number of times corresponding to the number I of patients included in the medical data corresponding to the above-described data range.

That is, the generation unit 15B generates a list of medical institutions for medical functions when an i-th patient uses medical services corresponding to the medical functions, for example, hyperacute phase, acute phase, recovery phase, and maintenance phase (step S103). Then, the generation unit 15B sorts the medical institutions included in the medical institution list for the i-th patient obtained by generating the list in step S103 in time series (step S104).

Thereafter, the generation unit 15B executes loop processing 2 that repeats processing from the following step S105 to the following step S109 by the number of times corresponding to the number J of medical institutions included in the medical institution list for the i-th patient.

That is, the generation unit 15B determines whether or not no node corresponding to a j-th medical institution among the J medical institutions included in the medical institution list for the i-th patient has been generated on the care pathway being generated (step S105).

Then, when no node corresponding to the j-th medical institution has been generated (Yes in step S105), the generation unit 15B adds a node corresponding to the **j-**th medical institution to the care pathway being generated (step S106).

Thereafter, the generation unit 15B determines whether or not no edge has been generated between the node of the j-th medical institution and the node of the j-1th medical institution in the care pathway being generated (step S107).

At this time, when no edge has been generated between the node of the j-th medical institution and the node of the j-1th medical institution (Yes in step S107), the generation unit 15B adds an edge between the node of the j-th medical institution and the node of the j-1th medical institution (step S108). Furthermore, the generation unit 15B increments the number of paths for the edge connecting the node of the j-th medical institution and the node of the j-1th medical institution, that is, the number of patients, by one (step S109).

By repeating such loop processing 2, a care path for the i-th patient is generated. Further, by repeating the loop processing 1, a care pathway in which care paths for I people are combined is generated.

### (2) Calculation Processing

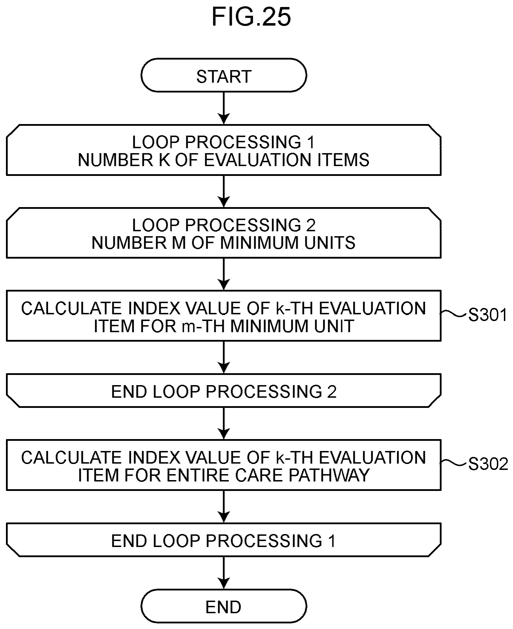

FIG. 25 is a flowchart illustrating a procedure of calculation processing. As illustrated in FIG. 25, the calculation unit 15C executes loop processing 1 that repeats processing in the following step S301 and processing in the following step S302 by the number of times corresponding to the number K of evaluation items. Furthermore, the calculation unit 15C executes loop processing 2 that repeats the processing in the following step S301 for the minimum unit corresponding to the k-th evaluation item. That is, the calculation unit 15C calculates an index value of the k-th evaluation item for an m-th minimum unit (step S301). By repeating such loop processing 2, index values of the k-th evaluation item for M minimum units are calculated. Thereafter, the calculation unit 15C calculates an index value of the k-th evaluation item for the entire care pathway by calculating a statistical value, for example, an average value, of index values of the k-th evaluation item for M minimum units (step S302). Then, by repeating the loop processing 1, index values for the M minimum units and an index value for the entire care pathway are calculated for each of the K evaluation items.

### <One Aspect of Effect>

As described above, the server device 10 according to the present embodiment generates and displays a pathway indicating a route through medical institutions for medical functions when a plurality of users use medical services corresponding to the medical functions. Therefore, the server device 10 according to the present embodiment is capable of improving the visibility of the service route, for example, the cooperation state between the medical functions.

Furthermore, the server device 10 according to the present embodiment displays a care path and an index value for a specific individual in a superimposed manner on a care pathway indicating a route through medical institutions for medical functions when a plurality of users use medical services corresponding to the medical functions. Therefore, the server device 10 according to the present embodiment is capable of verifying the effectiveness of measure for a specific user.

Furthermore, the server device 10 according to the present embodiment displays nodes corresponding to the same type of medical function in a common display format between a measure flow and a care pathway, and displays medical institutions included in the care pathway in association with their locations on the map. Therefore, the server device 10 according to the present embodiment is capable of improving the readability of the measure flow.

### <Second Embodiment>

Although the embodiment relating to the disclosed device has been described so far, the present invention may be embodied in various different forms other than the above-described embodiment. Therefore, other embodiments that fall within the present invention will be described below.

### <Distribution and Integration>

In addition, it is not required that each component of each device illustrated in the drawings be physically configured as illustrated in the drawings. That is, the specific distributed and integrated form of each device is not limited to the illustrated form, and all or part of each device can be functionally or physically distributed and integrated in any unit depending on various loads, usage conditions, and the like. For example, the reception unit 15A, the generation unit 15B, the calculation unit 15C, or the display unit 15D may be connected via a network as an external device of the server device 10. In addition, the reception unit 15A, the generation unit 15B, the calculation unit 15C, or the display unit 15D may be provided in a separate device connected via a network for cooperation to implement the function of the server device 10.

### <Hardware Configuration>

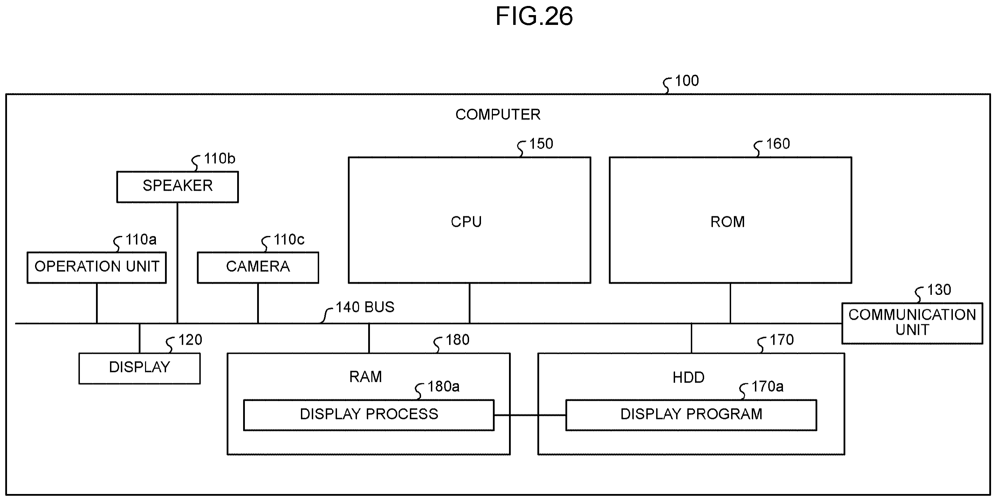

In addition, the various types of processing described in the above embodiments can be realized by executing a program prepared in advance on a computer such as a personal computer or a workstation. Therefore, an example of a computer that executes a display program having the same functions as those in the first and second embodiments will be described below with reference to FIG. 26.

FIG. 26 is a diagram illustrating an example of a hardware configuration. As illustrated in FIG. 26, the computer 100 includes an operation unit 110a, a speaker 110b, a camera 110c, a display 120, and a communication unit 130. The computer 100 further includes a CPU 150, a ROM 160, an HDD 170, and a RAM 180. These units 110 to 180 are connected to each other via a bus 140.

As illustrated in FIG. 26, the HDD 170 stores a display program 170a that exerts functions similar to those of the reception unit 15A, the generation unit 15B, the calculation unit 15C, and the display unit 15D described in the first embodiment described above. The display program 170a may be integrated or separated similarly to the components of the reception unit 15A, the generation unit 15B, the calculation unit 15C, and the display unit 15D illustrated in FIG. 1. That is, it is not necessary that the HDD 170 store all the data described in the first embodiment described above, as long as data to be used for processing may be stored in the HDD 170.

Under such an environment, the CPU 150 reads the display program 170a from the HDD 170, and then loads the display program 170a into the RAM 180. As a result, as illustrated in FIG. 26, the display program 170a functions as a display process 180a. The display process 180a loads various types of data read from the HDD 170 into an area assigned to the display process 180a among storage areas included in the RAM 180, and executes various types of processing using the loaded various types of data. For example, the processing illustrated in FIGS. 24 and 25 is included as an example of the processing executed by the display process 180a. Note that, in the CPU 150, it is not necessary that all the processing units described above in the first embodiment operate, as long as a processing unit corresponding to processing to be executed is virtually realized.

Note that it is not necessary that the display program 170a be stored in the HDD 170 or the ROM 160 from the beginning. For example, each program is stored in a "portable physical medium" such as a flexible disk inserted into the computer 100, a so-called FD, CD-ROM, DVD disk, magneto-optical disk, or IC card. Then, the computer 100 may acquire and execute each program from the portable physical medium. In addition, each program may be stored in another computer, a server device, or the like connected to the computer 100 via a public line, the Internet, a LAN, a WAN, or the like, and the computer 100 may acquire and execute each program therefrom.

### Reference Signs List

10 SERVER DEVICE
11 COMMUNICATION CONTROL UNIT
13 STORAGE UNIT
13A MEDICAL DB
13B MEASURE DB
15 CONTROL UNIT
15A RECEPTION UNIT
15B GENERATION UNIT
15C CALCULATION UNIT
15D DISPLAY UNIT
30 CLIENT TERMINAL

## Claims

1. A display method executed by a computer, the display method comprising:
displaying a path indicating a relationship diagram between services in different colors for respective types of services in a first area of a screen in a format including conditional branches; and
changing a display position of the path with respect to a map such that display positions of institutions providing the services included in the path are superimposed on the map in a second area of the screen, and displaying the map and the path with the services in the different colors.

2. The display method according to claim **1,** further comprising:
when another path different from the path and another map corresponding to the another path are acquired,
displaying the another path in a format including conditional branches, with services included in the another path in different colors, in the first area; and
changing a display position of the another path with respect to the another map such that display positions of institutions providing the services included in the another path are superimposed on the another map in the second area, and displaying the another map and the another path with the services included in the another path in the different colors.

3. The display method according to claim **2,** further comprising:
when an index for each of the path and the another path is acquired, displaying the index for each of the path and the another path in a third area of the screen such that the index for the another path is emphasized with respect to the index for the path.

4. The display method according to claim 1, further comprising:
when user information is acquired for a user who uses the services, calculating route information for the user in the flow based on a learning model that has learned a relationship between user information for each of a plurality of users and the services; and
displaying a route for the user in an emphasized manner in the path displayed in the second area using the calculated route information.

5. The display method according to claim 4, further comprising:
when the route information is calculated, referring to a storage unit that stores index values related to an evaluation item for the services, and calculating an index value related to the evaluation item for the user using the route information; and
displaying the calculated index value related to the evaluation item for the user on the screen.

6. An information processing apparatus comprising a control unit that executes:
displaying a path indicating a relationship diagram between services in different colors for respective types of services in a first area of a screen in a format including conditional branches; and
changing a display position of the path with respect to a map such that display positions of institutions providing the services included in the path are superimposed on the map in a second area of the screen, and displaying the map and the path with the services in the different colors.

7. The information processing apparatus according to claim 6, wherein the control unit further executes:
when another path different from the path and another map corresponding to the another path are acquired,
displaying the another path in a format including conditional branches, with services included in the another path in different colors, in the first area; and
changing a display position of the another path with respect to the another map such that display positions of institutions providing the services included in the another path are superimposed on the another map in the second area, and displaying the another map and the another path with the services included in the another path in the different colors.

8. The information processing apparatus according to claim 7, wherein the control unit further executes:
when an index for each of the path and the another path is acquired, displaying the index for each of the path and the another path in a third area of the screen such that the index for the another path is emphasized with respect to the index for the path.

9. The information processing apparatus according to claim 6, wherein the control unit further executes:
when user information is acquired for a user who uses the services, calculating route information for the user in the flow based on a learning model that has learned a relationship between user information for each of a plurality of users and the services; and
displaying a route for the user in an emphasized manner in the path displayed in the second area using the calculated route information.

10. The information processing apparatus according to claim 9, wherein the control unit further executes:
when the route information is calculated, referring to a storage unit that stores index values related to an evaluation item for the services, and calculating an index value related to the evaluation item for the user using the route information; and
displaying the calculated index value related to the evaluation item for the user on the screen.

11. A display program causing a computer to execute:
displaying a path indicating a relationship diagram between services in different colors for respective types of services in a first area of a screen in a format including conditional branches; and
changing a display position of the path with respect to a map such that display positions of institutions providing the services included in the path are superimposed on the map in a second area of the screen, and displaying the map and the path with the services in the different colors.

12. The display program according to claim 11, wherein the computer is caused to further execute:
when another path different from the path and another map corresponding to the another path are acquired,
displaying the another path in a format including conditional branches, with services included in the another path in different colors, in the first area; and
changing a display position of the another path with respect to the another map such that display positions of institutions providing the services included in the another path are superimposed on the another map in the second area, and displaying the another map and the another path with the services included in the another path in the different colors.

13. The display program according to claim 12, wherein the computer is caused to further execute:
when an index for each of the path and the another path is acquired, displaying the index for each of the path and the another path in a third area of the screen such that the index for the another path is emphasized with respect to the index for the path.

14. The display program according to claim 11, wherein the computer is caused to further execute:
when user information is acquired for a user who uses the services, calculating route information for the user in the flow based on a learning model that has learned a relationship between user information for each of a plurality of users and the services; and
displaying a route for the user in an emphasized manner in the path displayed in the second area using the calculated route information.

15. The display program according to claim 14, wherein the computer is caused to further execute:
when the route information is calculated, referring to a storage unit that stores index values related to an evaluation item for the services, and calculating an index value related to the evaluation item for the user using the route information; and
displaying the calculated index value related to the evaluation item for the user on the screen.
